# EUROPEAN PATENT SPECIFICATION

(11) **EP 1 856 516 B1**
(45) Date of publication and mention of the grant of the patent: **09.05.2012**
(21) Application number: 05715983.2
(22) Date of filing: 11.03.2005
(51) Int. Cl.: G01N 27/48, G01N 27/49

(54) **METHOD AND DEVICE FOR THE ELECTROCHEMICAL PSEUDO-TITRATION OF ANTIOXIDANT SUBSTANCES**
VERFAHREN UND VORRICHTUNG ZUR ELEKTROCHEMISCHEN PSEUDOTITRATION VON ANTIOXIDANTIEN
PROCEDE ET DISPOSITIF POUR LE PSEUDOTITRAGE DE SUBSTANCES ANTIOXYDANTES

(43) Date of publication of application: 21.11.2007
(73) Proprietor: Edel Therapeutics S.A., 1015 Lausanne (CH)
(72) Inventor: LAGGER, Grégoire, CH-1205 Geneva (CH); TACCHINI, Philippe, CH-1202 Geneva (CH); GIRAULT, Hubert H., CH-1015 Lausanne (CH)
(74) Representative: Becker Kurig Straus
(86) International application number: PCT/EP2005/002623
(87) International publication number: WO 2006/094529

(56) References cited:
- WO-A-96/13193
- WO-A-2004/044576
- US-A- 5 239 258
- US-B1- 6 638 415
- S. CHEVION, M.A. ROBERTS, M. CHEVION: "The use of cyclic voltammetry for the evaluation of antioxidant capacity" FREE RADICAL BIOLOGY & MEDICINE, vol. 28, no. 6, 2000, pages 860-870, XP002351858 USA
- E.I. KOROTKOVA, Y.A. KARBAINOV, A.V. SHEVCHUK: "Study of antioxidant properties by voltammetry" J. ELECTROANAL. CHEMISTRY, vol. 518, 2002, pages 56-60, XP002351859
- A.J. BLASCO, M.C. GONZALEZ, A. ESCARPA: "Electrochemical approach for discriminating and measuring predominant flavonoids and phenolic acids using differential pulse voltammetry: towards an electrochemical index of natural antioxidants" ANALYTICA CHIMICA ACTA, vol. 511, 2004, pages 71-81, XP002351860
- J.PSOTOVA, J. ZAHALKOVA, J. HRBAC, V. SIMANEK, J. BARTEK: "Determination of total antioxidant capacity in plasma by cyclic voltammetry. Two case reports" BIOMED. PAPERS, vol. 145, no. 2, 2001, pages 81-83, XP002351861
- P.A. KILMARTIN: "Electrochemical detection of natural antioxidants: principles and protocols" ANTIOXIDANTS & REDOX SIGNALING, vol. 3, no. 6, 2001, XP008054904

## Description

### Field of the invention

The present invention relates to the electrochemical titration, detection or identification of antioxidant substances and related devices.

### Background of the invention

Electrochemical sensors are used in many analytical devices for the measurement of numerous compounds. The electro analytical determination of antioxidant molecules, by measuring, for example, their oxidation current as a function of the applied potential has also extensively been demonstrated.
US-6,638,415 describes a device for measuring the level of oxidant or antioxidant analytes in a fluid sample. The device consists of a disposable electrochemical cell containing a reagent capable of undergoing a redox reaction with the analyte. Heat may be applied by a resistive heating element or by an exothermic material contained within the cell, in case slow reacting analytes are to be used.
WO/2004044576 describes a method for determining the oxidant/antioxidant activity of a substance based on the addition of a mediator to the substance to be analyzed and US-4,155,713 describes an assay for measuring the antioxidant concentration in hydrocarbonaceaous materials based on the addition of specific peroxy radicals.
US 5,239,258 discloses a method for analyzing the oxidative degradation of organic material like e.g. fuels, food products, oils or lubricants. Accordingly, a sample is subject to sweep voltametric analysis to measure the current through the sample as a function of the potential applied. The resulting current-voltage plot reflects the level of oxidation products present in the sample.
Chevion et al. in Free Radical Biology & Medecine Vol 28, pp 860 - 870 (2000) discloses a classical electrochemical method (cyclic voltammetry) for evaluating the antioxidant capacity of low-molecular weight antioxidants. The various parameters which more or less relate to the antioxidant capacity of the tested sample are readily obtained from the voltammogram and the oxidation potential is expressed in Volt or mVolt whereas the oxidation current is expressed in Ampere or mAmpere.
Available electrochemical methods either rely on the addition of electrochemically active mediators and/or radicals and do not allow the measurement of antioxidant power of the tested samples without physically and/or chemically modifying it prior its analysis or rely on an unprocessed electrochemical signature.
An object of the present invention resides in obviating the shortcomings of prior art and to provide a new means for measuring antioxidant power by pseudo-titration and with the ability to take in-situ measurement in solution, in biological samples, including on wet tissues.

### The Invention

This invention refers to a method for the indirect electrochemical measurement, in fact a pseudo-titration of the antioxidant power of antioxidant substances, the use of this method for the identification and/or detection and/or titration of antioxidant substances in a tested material, including directly on wet biological tissues and a device for performing the described method.

In particular the invention refers to a method for the indirect electrochemical measurement of the antioxidant power of an antioxidant substance which comprises the steps of:
- placing a sample of a material containing at least one antioxidant substance in contact with a sensor comprising at least one working electrode, one reference electrode and one auxiliary electrode to afford an electrochemical signal;
- applying a predefined potential waveform to the sensor while measuring the variation of the electrochemical signal between the working and the auxiliary electrode(s) to afford a primary signal;
- modulating the primary signal by applying a mathematical function representing the titration of an ideal oxidizing agent;
- reading the antioxidant activity as an antioxidant power by integrating the obtained current signal over the applied potential.

The invention further refers to a device useful for performing the method defined here above, which consists of an electrochemical unit and an electrochemical sensor for measuring antioxidant power which is expressed in antioxidant power units, comprising:
- at least one mono- or multi- surface working electrode;
- a potentiostat; and
- electronic processors for treating the electrochemical signal and generating the final signal output expressed in antioxidant power units.

### Brief description of the Drawings

Fig. 1 illustrates pseudo-titration curves for the saliva of three different patients;
Fig. 2 illustrates curves corresponding to modulating titration functions;
Fig. 3 is a schematic representation of a multi-surface working electrode;
Fig. 4 is a schematic representation of a system comprising three electrodes;
Fig. 5 illustrates the direct testing on a patient.

### Detailed description of the invention

The invention consists of generating an electrochemical signature of the analyzed substances, obtained by carrying out a numerical pseudo-titration. The principle of which is the processing of a current-potential response of the oxidation of the analyzed substances by a predefined mathematical function representing a virtual and ideal oxidizing agent.

The device consists of an electrochemical unit and an electrochemical sensor for generating the electrochemical signature of the analyzed antioxidant, including at least one mono or multi surface electrode, with or without an extension arm, a potentiostat and electronic processors for treating the electrochemical signal and generating the final signal output expressed in power units.

According to a first embodiment of the invention the sample comprising or supposed to comprise one or several antioxidant substances can be analyzed according to the method described here below.
The method consists in the progressive electrochemical oxidation, typically from 0 to 1 V, of the antioxidant molecules present in the solution. The oxidation takes place onto an electrode surface specially designed to allow several types of molecules to react. The working electrode, where these oxidations take place is a composite material resulting from the assembly of different types of surfaces. For example, activated carbon surfaces are particularly well adapted for neutral hydrophilic compounds, while gold ones, covered by an organic gel, allows the oxidation of more hydrophobic molecules. The progressive oxidation is conducted using an optimized electrochemical signal such as, e.g., a differential pulse polarography adapted to solid surfaces. This original signal is applied, via a potentiostat to a system of working and reference electrodes, with or without a counter (or auxiliary) electrodes. The resulting signal is recovered as current changes in function of the applied potential and represents the primary signal, which is then processed. Indeed, antioxidant molecules that have a measured oxidation potential above a reference value, taken at 0.5 V for the reduced glutathione (e.g.), have to be discriminated compared to those who have a lower oxidation potential. The oxidation potential of reference can be adjusted from 0 to 1 V, depending on the tested solution. This signal treatment corresponds to an electrochemical pseudo-titration of a mixture of antioxidants, against a given reference antioxidant. This mathematical treatment is based on Fermi-Dirac function and includes other monotonous decreasing dimensionless function. The sum of each oxidation current per potential increment corresponds to the antioxidant power of the solution: it can be expressed either in electrical power units (Watt) or in any other specific unit such as an antioxidant power (AOP). Pouvoir AntiOxydant (PAOx), or in total antioxidant power (TAO), Pouvoir AntiOxydant Total (TAOx) or any other suitable unit.

According to a specific embodiment of the invention, the auxiliary electrode and the reference electrode can be combined in one electrode assuming both functions.

According to another specific embodiment of the invention, the signal mentioned here above can be generated by means of several working electrodes used simultaneously and/or sequentially and/or successively, for the electrochemical oxidation of different antioxidant molecules. Alternatively, the signal can be generated by a single working electrode comprising several different surfaces used simultaneously and/or sequentially and/or successively for the electrochemical oxidation of different antioxidant molecules.

According to the present invention the device integrates a set of electrochemical sensors, an electrical signal generator and a potentiostat, an electronic signal processing, including filters and integrators and a read out for measuring the antioxidant power of the solution. The electrochemical sensors design may include an extension arm and a protective coating for allowing its use directly on a variety of solutions, including e.g. biological fluids on wet tissue.

A further embodiment of the invention consists in a method for fabricating such a sensor and the use of such a sensor as well, for the direct analysis of antioxidant power. For the preparation of the combined electrode, conductive and electro active particles are provided, which may be made of any suitable conductive material, such as carbon (graphite), gold and/or platinum or a combination thereof. In general, the particles have the shape of flakes or balls, and exhibit a size of between 0.01 and 500 µm, preferably between 1 and 20 µm. Particles can also be mixed with or replaced by colloids, in which case the size ranges of from 0.001 and 1 µm.
Organic molecules, such as peptides, proteins and/or nucleic acids can be added to these particles. The peptide and protein mass comprises molecules from 660 to 100'000 Daltons, while nucleic acid include double and single strand DNA and RNA molecules ranging from single nucleotide to several millions nucleotides and their combination.
If desired, the obtained particles and/or colloids may be treated by physical or chemical means, such as laser or plasma irradiation, by mechanical grinding, laminating or heat (pyrolitic) treatment or with oxidizing, acidifying or bonding agents, such as e.g. ferrocene carboxylic acid, so as to make the sensor more selective, sensitive and to specify the dynamic range of the analysis when used as an electrochemical device.
The obtained material is molded to any shape or deposited onto a suitable substrate/support in thin or thick layers in conventional ways, such as by printing, leading to a final sensor volume on which electrochemical, chemical and other reactions may be conducted. As a substrate/support, any non-conductive or also conductive material may be used, such as cellulose, polyester, polystyrene, metal, electrode, organic tissues. The obtained surface is then recovered by any protective coating so that diffusion onto the working surface can be controlled and the working surface protected from contaminants. Such film might consist of inert or active materials such as plastic and/or specific gel and/or organic molecules, including peptides, proteins and nucleic acids.
All shapes of dried material may be obtained. The typical thickness of the dried layer ranges from 1 to several hundreds micrometers, preferably of from 2 to 50 µm. The temperature selected for drying is preferably in the range of 30° to 50°C, and the time period is from several seconds to several hours, preferably from about 1 minute to about 15 minutes.
Depending on the solvent added to the mixture and the drying condition, the resulting composite material can be porous or non-porous.
The material may be exposed directly to the substance to be analyzed and/or is available for further treatments. These treatments include mechanical polishing, light irradiation at any wavelength, UV, X-ray, photon treatments, other radioactive activations such as with alpha-, beta-particles or neutrons, chemical activation such as acidic or basic treatment, oxidation, electrochemical activation such as reduction, oxidation, biological, biochemical treatments or combination of these techniques.
All treatments may also be conducted at specific, geometrically well defined locations on the surface of the sensor. In addition, other insulating or conductive materials, such as polymeric solutions, metallic layers, inks, glues, solvents, etc. may be deposited onto certain locations/regions/areas on the surface. Thus, a patterning of the surface of the sensor may be obtained. The area of the sensor may be controlled by a first printing or molding stage, by the adding of layers geometrically defined that can be of different mixture compositions. Any step of printing, molding or treatment may be repeated in all kinds of sequences.
The sensor is connected as the working electrode into an electrochemical cell comprising a reference electrode such as a silver/silver chloride wire or layer and a counter electrode such as a platinum, gold, carbon wire or layer.
When using the sensor obtainable according to the above described method steps for determining a desired entity, the sensor is placed in contact with the sample to be analyzed, i.e. a sample suspected to modify and/or alter, e.g. oxidize the combined sensor. In general, the sample may be in any form allowing contact with the sensor, e.g. the form of a solution, a gas or even in solid form.
Repeated electrochemical signals can be recorded over time and give an indication of the reaction effect dynamics.
The principle of the treatment of the signal is the following: the obtained primary signal is treated with a specific filter which attributes coefficients to the original measure, so that an integrated signal can be generated. Simultaneously, a discrete measurement is also taken, allowing the establishment of internal control and/or internal reference value. These references might include any molecules harboring a specific electrochemical signature, such as, for example, single nucleotides and/or polynucleotides, nucleic acids, carboxylic acids, such as the reduced ferrocene salt.
This results in the possibility to characterize a given antioxidant, or a mixture of antioxidants in e.g. liquids, gels and gases. Applications include the analysis of any substance capable of holding antioxidant molecules including food, beverages, drugs, environments, liquids, perfusion products, biological fluids such as saliva, blood, serum, plasma, urine tears, sweat, inter- and intra cellular fluids, etc. Moreover, such sensor design and fabrication method allows it to be used directly onto biological tissues, allowing a direct and real time measurement of oxidant/antioxidant activity contained in such biological solutions. In particular, this sensor might be used for the antioxidant power determination onto oral wet tissue (tongue) and/or wet skin (sudation) and bleeding tissues (blood).

### Examples

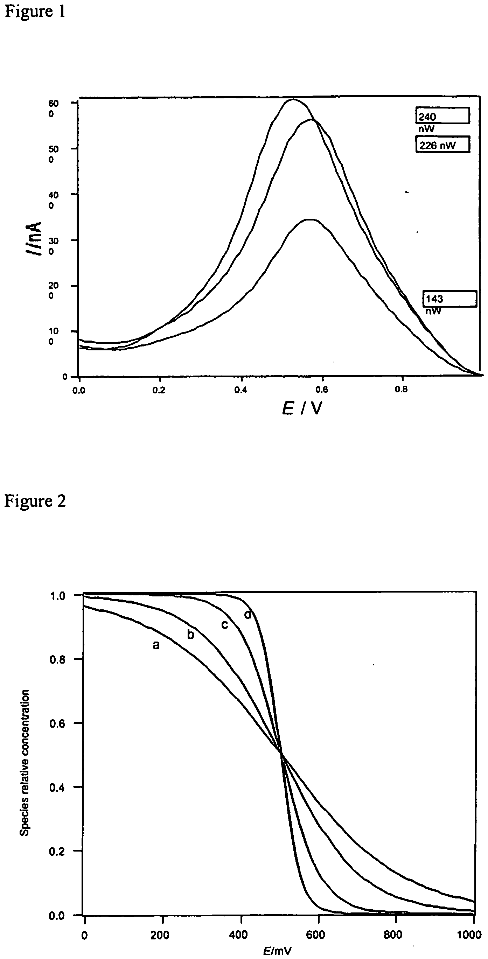

Examples of resulting antioxidant power signals of the saliva of three patients have been obtained according to the above; they are shown in Figure 1. Curves a and b refer to healthy patients (resulting antioxidant power of 240 and 226 nW respectively) while curve c refers to a patient with a depressed antioxidant system (antioxidant power of 143 nW).

The pseudo-titration signals applied in the method described here above can be shown in Figure 2. Curves a, b, c and d correspond to the equivalent reduced proportion of the reference antioxidant having, in this case, a redox potential of 500 mV.

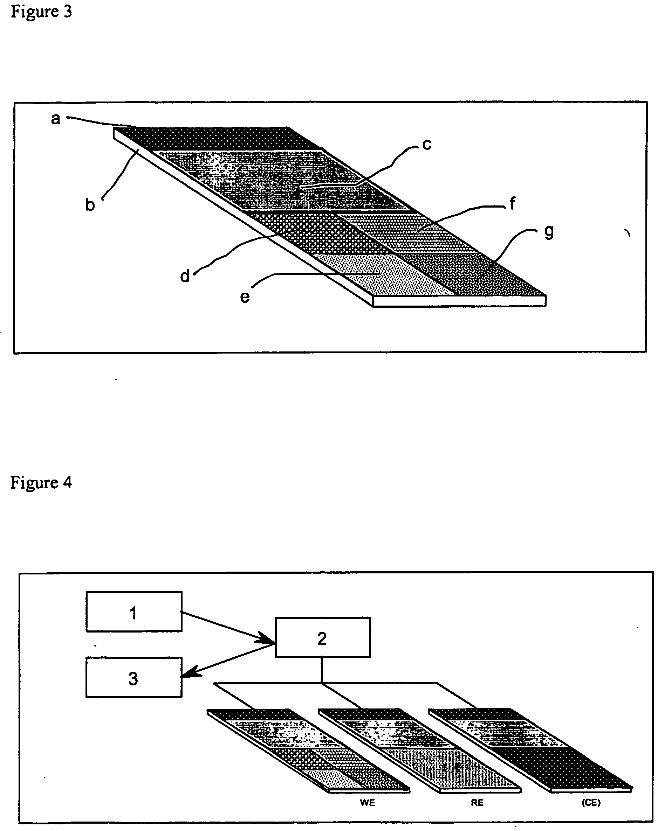

The working electrode used to apply the method described here above is represented in Figure 3. Such an electrode usually consists of a conducting track made of any conducting material such as a dry carbon ink (a) deposited onto a non-conducting substrate such as any polymer material (b). An insulating layer (c) is usually deposited to define a sensing area. This sensing area can consist in the assembly of different surface materials (d, e, f, g), providing different physical-chemical properties to the solution molecules, in order to maximize the number of species able to undergoing redox reactions.

Figure 4 is a schematic representation of the device described here above with the signal generator (1), the potentiostat (2), the signal processor (3) (with electronic filters and pseudo-titration integrator) and output; WE, RE and CE refer to the working electrode, the reference electrode and the counter (or auxiliary) electrode, respectively.

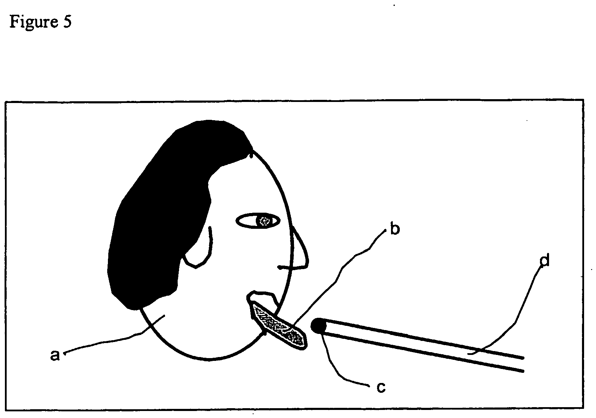

The electrodes can be placed at the end of a collecting arm as shown in Figure 5. A patient (a) can have his/her saliva tested in the mouth or on the tongue (b) or any wet tissue by simply contacting the top end - where are placed the electrodes (c) - of the collecting arm (d). This arm can be made of polymer and can hold the entire device or only the connection cables from the electrodes at the top end to the signal generator, potentiostat and signal processing unit that can be placed outside the arm.

## Claims

1. A method for the indirect electrochemical measurement of the antioxidant power of an antioxidant substance which comprises the steps of:
- placing a sample of a material containing at least one antioxidant substance in contact with a sensor comprising at least one working electrode, one reference electrode and one auxiliary electrode to afford an electrochemical current signal;
- applying a predefined potential waveform to the sensor between two potential values between which oxidation of the antioxidant substance occurs while measuring the variation of the electrochemical current signal between the working and the auxiliary electrode(s) to afford a primary signal;
- *modulating* the primary signal by applying thereto a mathematical treatment based on a Fermi-Dirac function which simulates a virtual electrochemical titration of a reference molecule which oxidation potential ranges from 0 to 1.5 V and which includes any monotonous decreasing dimensionless function between one and zero; and
- reading the antioxidant activity as an antioxidant power, expressed in electrical power units or in specific units such as an antioxidant power units, by integrating the *modulated* primary current signal over the applied potential.

2. The method according to claim 1, wherein the auxiliary electrode and the reference electrode are combined in one electrode assuming both functions.

3. The method according to claim 1, wherein the signal is generated by several working electrodes used simultaneously and/or sequentially and/or successively for the electrochemical oxidation of different antioxidant molecules.

4. The method according to claim 1, wherein the signal is generated by a single working electrode comprising several different surfaces used simultaneously and/or sequentially and/or successively for the electrochemical oxidation of different antioxidant molecules.

5. The method according to any of claims 1 to 4, wherein the sensor is in contact with the solution directly on wet biological tissue.

6. The use of the method according to any of claims 1 to 5 for the identification and/or detection and/or titration of antioxidant substances.

7. A device for the indirect electrochemical measurement of the antioxidant power of an antioxidant substance which consists of an electrochemical unit and an electrochemical sensor for measuring the antioxidant power and expressing the latter in antioxidant power units, the sensor comprising:
- at least one mono- or multi- surface working electrode;
and the unit comprising:
- a potentiostat;
- electronic processors arranged for treating the electrochemical current signal according to the method of claim 1 and generating the final signal output expressed in electrical power units or specific units such as an antioxidant power units.

8. The device according to claim 7, wherein each working electrode surface is made of any conductive particles, including carbon, metallic particles and colloids, oxides or their combination.

9. The device according to claim 7, wherein the sensor includes organic molecules, indicators or dyes.

10. The device according to claim 9, wherein organic molecules including peptides and/or proteins from 660 to 100'000 Daltons and single or double strand DNA and RNA molecules from single nucleotide to several millions nucleotides and their combination, are added to or deposited onto the conductive particles and/or the obtained electrode surface.

11. The device according to claim 7, wherein each working electrode surface is pre-treated by any physical or chemical means such as laser or plasma irradiation, mechanical grinding laminating, heating, oxidizing, acidifying or bonding agents such as ferrocene carboxylic acid.

12. The device according to claim 7, wherein each working electrode surface is covered by a grid made of a porous material such as cellulose or polymer, or by an aqueous or organic gel or an organic substance.

13. The device according to claim 7 wherein a protective coating is applied to the sensor surface, including inert or active coating consisting of polymer, ceramic, cellulose and/or the combination thereof

14. The device according to any of the preceding claims, wherein the electrode material is deposited onto a substrate consisting of an isolating material, such as paper or polymers.

15. The device according to any of the preceding claims, wherein the working and/or the reference electrode is printed, layered, embedded or engraved onto the above substrates, or is molded or injected with or without substrate onto a specific shape, at temperatures ranging from -230°C to 400°C.

16. The device according to any of the preceding claims, wherein the sensor is placed at the top end of an extension arm.

17. The device according to claim 16, wherein the extension arm allows to hold and to position the sensors in contact with the surface to be tested.

## Patentansprüche

1. Verfahren zur indirekten elektrochemischen Bestimmung der antioxidativen Befähigung einer antioxidativen Substanz, umfassend die Schritte:
- In Kontakt Bringen einer Materialprobe, welche mindestens eine antioxidative Substanz umfasst, mit einem Sensor, welcher mindestens eine Arbeitselektrode, eine Referenzelektrode und eine Hilfselektrode umfasst, um ein elektrochemisches Stromsignal bereitzustellen;
- Anwenden einer bestimmten Potenzialwellenform auf den Sensor zwischen zwei Potentialwerten, zwischen welchen Oxidation der antioxidativen Substanz auftritt, während einer Bestimmung der Änderung des elektrochemischen Stromsignals zwischen der Arbeits- und der(den) Hilfselektrode(n), um ein erstes Signal bereitzustellen;
- Modulieren des ersten Signals durch Anwenden eines mathematischen Verfahrens basierend auf einer Fermi-Dirac Funktion darauf, welche eine virtuelle elektrochemische Titration eines Referenzmoleküls simuliert dessen Oxidationspotenzial im Bereich von 0 bis 1,5 V liegt und welches irgendeine monoton abnehmende, dimensionslose Funktion zwischen Eins und Null umfasst; und
- Erfassen der antioxidativen Aktivität als eine antioxidative Befähigung, welche in elektrischen Stromeinheiten oder in spezifischen Einheiten, wie beispielsweise antioxidativen Befähigungseinheiten, ausgedrückt ist, durch Integrieren des modulierten ersten Stromsignals über das angewendete Potential.

2. Verfahren nach Anspruch 1, worin die Hilfselektrode und die Referenzelektrode in einer Elektrode zusammengefasst sind, welche beide Funktionen annimmt.

3. Verfahren nach Anspruch 1, worin das Signal durch mehrere Arbeitselektroden erzeugt wird, welche gleichzeitig und/oder sequenziell und/oder nacheinander für die elektrochemische Oxidation verschiedener antioxidativer Moleküle verwendet werden.

4. Verfahren nach Anspruch 1, worin das Signal durch eine einzelne Arbeitselektrode erzeugt wird, welche mehrere verschiedene Oberflächen umfasst, welche gleichzeitig und/ oder sequenziell und/oder nacheinander für die elektrochemische Oxidation verschiedener antioxidativer Moleküle verwendet werden.

5. Verfahren nach einem der Ansprüche 1 bis 4, worin der Sensor mit der Lösung direkt auf feuchtem biologischem Gewebe in Kontakt steht.

6. Verwendung des Verfahrens nach einem der Ansprüche 1 bis 5, um antioxidative Substanzen zu identifizieren und/oder zu bestimmen und/oder zu titrieren.

7. Vorrichtung zur indirekten elektrochemischen Bestimmung der antioxidativen Befähigung einer antioxidativen Substanz, welche eine elektrochemische Einheit und einen elektrochemischen Sensor umfasst, um die antioxidative Befähigung zu bestimmen und die letztere in antioxidativen Befähigungseinheiten auszudrücken, wobei der Sensor umfasst:
- mindestens eine Arbeitselektrode mit Mono- oder Mehrfachoberfläche; und
wobei die Einheit umfasst:
- einen Potentiostaten;
- elektronische Prozessoren, welche zum Verarbeiten des elektrochemischen Stromsignals gemäß dem Verfahren nach Anspruch 1 und Erzeugen der Endsignalausgabe angeordnet sind, welche in elektrischen Stromeinheiten oder spezifischen Einheiten, wie beispielsweise antioxidativen Befähigungseinheiten, ausgedrückt ist.

8. Vorrichtung nach Anspruch 7, wobei jede Arbeitselektrodenoberfläche aus irgendwelchen leitfähigen Teilchen, einschließlich von Kohlenstoff, metallischen Teilchen und Kolloiden, Oxiden oder deren Kombination, hergestellt ist.

9. Vorrichtung nach Anspruch 7, wobei der Sensor organische Moleküle, Indikatoren oder Farbstoffe umfasst.

10. Vorrichtung nach Anspruch 9, wobei die organischen Moleküle Peptide und/oder Proteine von 660 bis 100.000 Dalton und einzel- oder doppelsträngige DNA und RNA Moleküle von einem Nukleotid bis zu mehreren Millionen Nukleotiden und deren Kombinationen umfassen, welche den leitfähigen Teilchen und/oder der erhaltenen Elektrodenoberfläche hinzugegeben oder darauf abgeschieden werden.

11. Vorrichtung nach Anspruch 7, wobei jede Arbeitselektrodenoberfläche durch irgendein physikalisches oder chemisches Mittel, wie beispielsweise Laser- oder Plasmabestrahlung, mechanisches Schleifen, Laminieren, Erhitzen, oxidierenden, versauernder, oder bindenden Agenzien, wie beispielsweise Ferrocencarboxylsäure, vorbehandelt ist.

12. Vorrichtung nach Anspruch 7, wobei jede Arbeitselektrodenoberfläche mit einem Netz bedeckt ist, welches aus einem porösen Material, wie beispielsweise Zellulose oder Polymer, oder durch ein wässriges oder organisches Gel oder einer organischen Substanz, hergestellt ist.

13. Vorrichtung nach Anspruch 7, wobei eine Schutzschicht auf die Sensoroberfläche aufgebracht ist, einschließlich einer inerten oder aktiven Beschichtung umfassend Polymer, Keramik, Zellulose und/oder Kombinationen davon.

14. Vorrichtung nach einem der vorstehenden Ansprüche, wobei das Elektrodenmaterial auf einem Substrat abgeschieden ist, welches ein Isoliermaterial, wie beispielsweise Papier oder Polymere, umfasst.

15. Vorrichtung nach einem der vorstehenden Ansprüche, wobei die Arbeits- und/oder die Referenzelektrode auf die vorstehenden Substrate gedruckt, beschichtet, eingebettet oder eingeprägt ist, oder mit oder ohne Substrat auf eine spezifische Form bei Temperaturen im Bereich von -230°C bis 400°C geformt oder eingespritzt ist.

16. Vorrichtung nach einem der vorstehenden Ansprüche, wobei der Sensor an dem oberen Ende eines Verlängerungsarms platziert ist.

17. Vorrichtung nach Anspruch 16, wobei der Verlängerungsarm ein Halten und Positionieren der Sensoren in Kontakt mit der zu testenden Oberfläche ermöglicht.

## Revendications

1. Un procédé pour la mesure électrochimique indirecte du pouvoir antioxydant d'une substance antioxydante qui comprend les étapes de :
- placer un échantillon de matière contenant au moins une substance antioxydante en contact avec un capteur comprenant au moins une électrode de travail, une électrode de référence et une électrode auxiliaire pour fournir une signal de courant électrochimique ;
- appliquer au capteur un signal d'onde de potentiel prédéfinie entre deux valeurs de potentiel entre lesquelles intervient l'oxydation de la substance antioxydante, tout en mesurant la variation du signal de courant électrochimique entre l'électrode de travail et (les)l'électrode(s) auxiliaire(s) pour fournir un signal primaire ;
- moduler le signal primaire en lui appliquant un traitement mathématique basé sur une fonction de Fermi-Dirac qui simule un titrage électrochimique virtuel d'une molécule de référence dont le potentiel d'oxydation s'échelonne de 0 à 1.5 Volts et qui inclut toute fonction sans dimension monotone décroissante entre 1 et zéro ; et
- lire l'activité antioxydante comme un pouvoir antioxydant exprimé en unités de puissance électrique ou en unités spécifiques telles que des unités de pouvoir antioxydant en intégrant le signal de courant primaire modulé sur le potentiel appliqué.

2. Le procédé selon la revendication 1 dans lequel l'électrode auxiliaire et l'électrode de référence sont combinées en une seule électrode exerçant les deux fonctions.

3. Le procédé selon la revendication 1 dans lequel le signal est généré par plusieurs électrodes de travail utilisées simultanément et/ou séquentiellement et/ou successivement pour l'oxydation électrochimique de molécules antioxydantes distinctes.

4. Le procédé selon la revendication 1 dans lequel le signal est généré par une électrode de travail unique comprenant plusieurs surfaces distinctes utilisées simultanément et/ou séquentiellement et/ou successivement pour l'oxydation électrochimique de molécules antioxydantes distinctes.

5. Le procédé selon l'une des revendications 1 à 4 dans lequel le capteur est directement en contact avec la solution sur du tissu biologique humide.

6. L'utilisation du procédé selon l'une des revendications 1 à 5 pour l'identification et/ou la détection et/ou le titrage de substances antioxydantes.

7. Un dispositif pour la mesure électrochimique indirecte du pouvoir antioxydant d'une substance antioxydante, qui consiste en une unité électrochimique et un capteur électrochimique pour mesurer le pouvoir antioxydant et exprimer ce dernier en unités de pouvoir antioxydant, le capteur comprenant :
au moins une électrode de travail mono ou multi surface ;
un potentiostat ;
des processeurs électroniques configurés pour traiter le signal de courant électrochimique selon le procédé de la revendication 1 et générer un signal de sortie final exprimé en unités de puissance électrique ou en unités spécifiques telles que des unités de pouvoir antioxydant.

8. Le dispositif selon la revendication 7 dans lequel chaque surface de l'électrode de travail est faite de toute particule conductrice comprenant le carbone, des particules et des colloïdes métalliques, des oxydes ou leur combinaison.

9. Le dispositif selon la revendication 7 dans lequel le capteur comprend des molécules organiques, des indicateurs ou des colorants.

10. Le dispositif selon la revendication 9 dans lequel dans lequel des molécules organiques comprenant des peptides et/ou des protéines de 660 à 100'000 Daltons et des molécules de brins d'ADN ou d'ARN simples ou doubles allant du simple nucléotide à plusieurs millions de nucléotides et leur combinaison sont ajoutées à ou déposées sur les particules conductrices et/ou la surface de l'électrode obtenue.

11. Le dispositif selon la revendication 7 dans lequel chaque surface d'électrode de travail est prétraitée par tout moyen physique ou chimique tel que irradiation au laser ou plasma, meulage mécanique, laminage, chauffage, oxydation, acidification ou au moyen d'agents de métallisation tel l'acide ferrocène carboxylique.

12. Le dispositif selon la revendication 7 dans lequel chaque surface d'électrode de travail est recouverte par une surface faite d'un matériau poreux tel que cellulose ou polymère ou par un gel aqueux ou organique ou une substance organique.

13. Le dispositif selon la revendication 7 dans lequel une couche protectrice est appliquée sur la surface du capteur, comprenant une couche inerte ou active consistant en polymère, céramique, cellulose et/ou la combinaison de ceux-ci.

14. Le dispositif selon l'une des revendications précédentes dans lequel la matière de l'électrode est déposée sur un substrat consistant en matériau isolant tel que papier ou polymères.

15. Le dispositif selon l'une des revendications précédentes dans lequel l'électrode de travail et/ou l'électrode de référence est imprimée, déposée, intégrée ou gravée sur les substrats ci-dessus, ou est moulée ou injectée avec ou sans substrat sur une forme spécifique, à des température s'étendant de -230° C à 400° C.

16. Le dispositif selon l'une des revendications précédentes dans lequel le capteur est disposé à l'extrémité sommitale d'un bras d'extension.

17. Le dispositif selon la revendication 16 dans lequel le bras d'extension permet de tenir et de positionner les capteurs en contact avec la surface à tester.
